# EUROPEAN PATENT SPECIFICATION

(11) **EP 1 548 438 B1**
(45) Date of publication and mention of the grant of the patent: **10.09.2008**
(21) Application number: 03799124.7
(22) Date of filing: 24.09.2003
(51) Int. Cl.: G01N 33/543, G01N 27/00

(54) **HYBRIDISATION DETECTING METHOD AND BIOASSAY DEVICE, AND BIOASSAY-USE SUBSTRATE**
HYBRIDISIERUNGSNACHWEISVERFAHREN UND BIOASSAY-VORRRICHTUNG UND BIOASSAY-VERWENDUNGSSUBSTRAT
PROCEDE DE DETECTION DE HYBRIDISATION ET DISPOSITIF DE BIOTEST, ET SUBSTRAT D'UTILISATION DE BIOTEST

(30) Priority: 04.10.2002 JP 2002292861
(43) Date of publication of application: 29.06.2005
(73) Proprietor: Sony Corporation, Tokyo 141-0001 (JP)
(72) Inventor: MAMINE, Takayoshi, c/o SONY CORPORATION, Tokyo 141-0001 (JP); KINOSHITA, Takashi, c/o SONY CORPORATION, Tokyo 141-0001 (JP)
(74) Representative: Horner, David Richard
(86) International application number: PCT/JP2003/012168
(87) International publication number: WO 2004/031769

(56) References cited:
- EP-A- 1 202 046
- WO-A-00/66266
- WO-A2-00/53812
- WO-A2-98/50773
- JP-A- 2002 250 726
- US-A- 5 827 482
- US-B1- 6 183 970

## Description

The present invention relates to a hybridization detecting method, a bioassay device and a substrate for bioassay especially useful in the bioinformatics (life information science) field.

An integrated circuit substrate for bioassay, what is called a DNA chip or a DNA microarray (generally refer it to as a DNA chip, hereinafter) in which a prescribed DNA is extremely finely arranged by a microarray technique is currently used for the mutation of genes and SNPs (single nucleotide polymorphism) analysis, the analysis of gene expression frequency or the like. The integrated circuit substrate has begun to be utilized in a wide range including a drug discovery, a clinical diagnosis, pharmacogenomics, a forensic medicine and other fields.

In the DNA chip, many kinds and many DNA oligonucleotide chains, cDNA (complementary DNA), etc. are accumulated on a glass base or a silicon base. Thus, the DNA chip is characterized in that the inclusive analysis of an intermolecular interaction such as hybridization can be realized.

One example of an analyzing method by the DNA chip is briefly described below. To a DNA probe in a state of a solid phase on the glass base or the silicon base, a fluorescent probe dNTP is incorporated and PCR-amplified under the reverse transcription PCR (Polymerase Chain reaction) of mRNA (messenger RNA) extracted from cells, tissues, etc. to perform the hybridization on the base. Thus, a fluorometry is carried out by a prescribed detector.

Here, the DNA chip can be sorted to two types. In a first type, oligonucleotide is directly synthesized on a prescribed base by using a photolithography technique to which a semiconductor exposure technique is applied. A DNA chip produced by Affymetrix Co., Ltd. is typical of the DNA chip of this type. The DNA chip of the first type is disclosed in, for instance, Japanese patent Publication No. hei 4-505763.

On the other hand, a second type is also called a "Stanford system", and is formed in such a way that a split pin is used to partially pour a previously prepared DNA on a base to bring the DNA to a solid phase. The DNA chip of the second type has an integration degree lower than the DNA chip of the first type. However, in the DNA chip of the second type, a fragment of the DNA of about 1 kb can be advantageously changed to the solid phase. The DNA chip of the second type is disclosed in, for instance, Japanese Patent Publication No. hei 10-503841.

Further, in a pamphlet of International Laid Open No. 01/33226, a cantilever sensor technique based on a technical idea different from the above-described conventional DNA chip technique is proposed, in which a cantilever formed by using an MEMS (Micro Electro Mechanical System: micro-machine) is used.

One example of an analyzing method by the cantilever sensor described in the pamphlet of International Laid Open No. 01/33226 is briefly described below. A cantilever having a DNA probe in a state of a solid phase on an upper surface is irradiated with a laser beam. The cantilever is bent as a result of a hybridization reaction to measure the change of the intensity of reflected light in a light receiving part at a prescribed position. Thus, whether or not the hybridization reaction occurs is detected.

However, the above-described conventional DNA chip technique is a technique that detecting nucleotide chains or protein of the DNA probe or the like are brought to a solid phase (fixed) in a narrow detecting part of a two-dimensional base to accelerate an interaction between materials such as the hybridization reaction or an antigen-antibody reaction. Accordingly, the degree of freedom of a reaction product is spatially limited and a steric hindrance may be possibly generated upon reaction. Therefore, in the conventional DNA chip technique, the efficiency of an interaction is inconveniently low and much reaction time is undesirably necessary.

Further, in the conventional DNA chip technique, sample solution is dripped only on a prescribed spot part (a detecting part) on the base. There is no means for positioning a target material included in the sample solution relative to a detecting material fixed to the spot part.

On the other hand, in the above-described cantilever sensor technique, the target material included in the sample solution does not need to be advantageously previously marked with fluorescence. However, when the cantilever is highly integrated, a mechanical control that the surface of the cantilever is irradiated with a laser beam to receive reflected light is undesirably difficult.

International Patent Application Publication No WO-A-00/66266 discloses a bioassay device comprising a detecting part in which a micro-cantilever has single stranded DNA from a disease-associated gene attached thereto using conventional binding chemistry. A blood sample consisting of single stranded DNA is then flushed through the device and, if a disease associated gene is present in the sample, it will bind to a DNA string attached to the micro-cantilever. The cantilever is vibrated, for instance by way of an integrated piezoelectric layer, and a change in resonant frequency is employed to detect the attachment of sample material to the cantilever.

United States Patent No US-A-5 827 482 discloses a molecular detection device in the form of a transistor having a detecting molecular receptor (nucleotide) proximate to a gate of the transistor, in which binding of a target molecule to the molecular receptor is enhanced by applying to the gate, source or drain of the transistor a voltage that attracts the target molecule to the molecular receptor.

The present invention provides a hybridization detecting method according to claim 1 hereof and a bioassay device according to claim 4 hereof. According to the invention as defined in those claims, a high frequency, high voltage electric field is formed in the reaction area, the field being uneven in that the field is concentrated at the surface treated part of the cantilever with the result that the nucleotide chains tend to move towards the concentrated part.

The invention also provides a substrate for bioassay according to claim 9 hereof.

Preferred features of the invention are set forth in the claims dependent upon claims 1,4 and 9.

The present invention may be further understood from the following description of illustrative embodiments thereof shown in the accompanying drawings, in which:
Fig. 1 is a perspective view showing an external appearance of a substrate for bioassay used in a bioassay device in an embodiment.
Fig. 2 is a perspective view showing one enlarged cell detecting part of many cell detecting parts disposed on the substrate for bioassay.
Fig. 3 is a perspective view showing an enlarged cantilever protruding on a reaction area side of the cell detecting part.
Figs. 4A to 4D are views for explaining an example of manufacturing processes of the cantilever.
Fig. 5 is a view showing a method for detecting the change of the natural frequency of the cantilever.
Fig. 6 is a diagram showing a voltage waveform measured when the frequency of ac power is changed.
Fig. 7 is a perspective view showing another structure of the cantilever.
Fig. 8 is a view showing a vertical section of the cantilever at a position including a piezoelectric element.
Figs. 9A and 9B are explanatory views of another example of the substrate used in the bioassay device. Fig. 9A shows a plan view of the substrate viewed from above. Fig. 9B shows an enlarged plan view of an X part in Fig. 9A.

Now, a specific embodiment to which the present invention is applied will be described below in detail by referring to the drawings. In a bioassay device and an interaction detecting method thereof described in this embodiment, an end part of a detecting nucleotide chain (a detecting material) is previously fixed to an end face of a cantilever having a vibrator composed of a piezoelectric material disposed between counter electrodes. The change of the natural frequency of the cantilever due to the change of mass that is caused from a hybridization reaction of the detecting nucleotide chain and a target nucleotide chain (a target material) is employed to quantitatively detect the hybridization reaction.

Here, in this specification, the "nucleotide chain" means a polymer of phosphoric ester of nucleotide in which a purine base or a pyrimidine base is glycoside bonded to sugar. The nucleotide chain widely includes oligonucleotide including a DNA probe, polynucleotide, DNA having purine nucleotide polymerized with pyrimidine nucleotide (entire length or a fragment thereof), cDNA obtained by a reverse transcription (cDNA probe), RNA, etc.

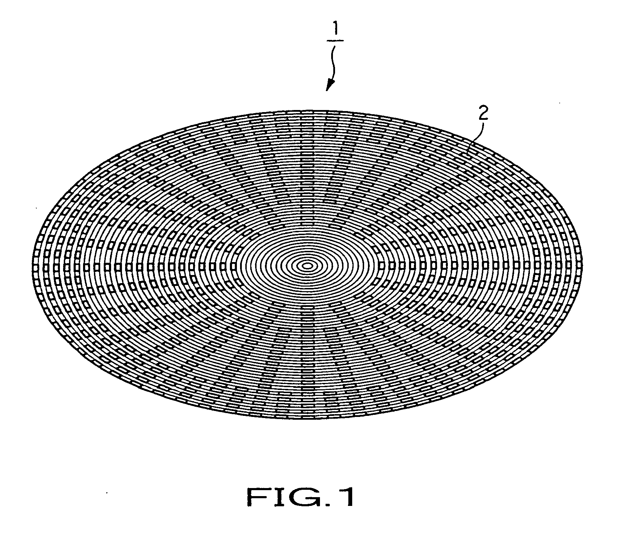

Firstly, a perspective view of an external appearance of a substrate for bioassay (abbreviate it as a substrate, hereinafter) 1 used in the bioassay device in this embodiment is shown in Fig. 1. The substrate 1 shown in Fig. 1 is formed with a base material employed for a disc type base used in an optical information recording medium such as a CD (Compact Disc), a DVD (Digital Versatile Disc), an MD (Mini Disc) (registered trademark).

The base material is formed to a disc shape by quartz glass, silicon, or a synthetic resin moldable in a disc shape such as polycarbonate, polystyrene, etc., and preferably by an injection-moldable synthetic resin. An inexpensive synthetic resin substrate is used, so that the substrate can be realized at a running cost lower than that of a conventional glass substrate. On the center of the substrate 1, a hole (not shown in the drawings) for fixing a spindle used for rotating the substrate may be formed.

On one surface of the substrate 1, many cell detecting parts 2 having small chamber type reaction areas partitioned from a peripheral substrate area are disposed so as to extend in radial directions from the peripheral area at the center of the substrate 1 viewed from above.

The cell detecting parts 2 may be disposed at proper positions on the substrate 1. However, the cell detecting parts 2 are disposed in the radial directions viewed from above as described above. Thus, a space on the substrate 1 can be effectively used to improve the integration density of information.

Further, the cell detecting parts 2 are partitioned not to be contaminated with each other. Thus, different detecting nucleotide chains can be fixed for units of the cell detecting parts or for units of the grouped cell detecting parts and separate and independent conditions can be set respectively for the detecting nucleotide chains to accelerate the hybridization reaction.

For instance, marker genes in which illness occurs can be grouped and fixed on the substrate 1. Accordingly, one substrate is used so that a plurality of illness appearing situations can be recognized at the same time.

Further, the detecting nucleotide chains to be fixed can be grouped on the basis of the difference of Tm (Melting Temperature) or the rate of content of GC. Thus, reaction conditions such as the composition, concentration or the like of a buffer from which an active hybridization reaction can be obtained, a cleaning condition, the concentration of dripped sample solution etc., can be finely selected in accordance with the property of the detecting nucleotide chains. Thus, a risk that a false positive or a false negative is shown in an analyzing operation can be reduced.

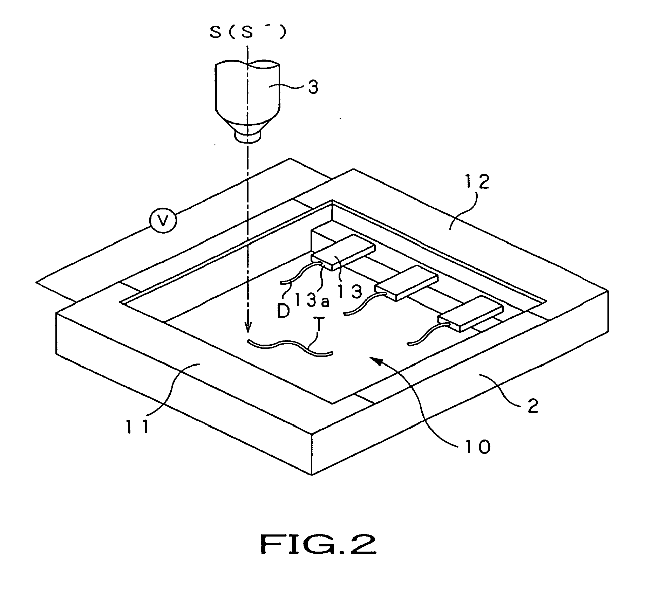

Fig. 2 shows a perspective view of an external appearance of one of the cell detecting parts 2 that is enlarged. As shown in Fig. 2, the cell detecting part 2 includes a reaction area 10 as a place of the hybridization reaction between a detecting nucleotide chain D and a target nucleotide chain T, a cathode 11 and an anode 12 for forming electric fields at both the ends of the reaction area 10, and a cantilever 13 protruding from the anode 12 side of the cell detecting part 2.

The reaction area 10 is formed as a substantially square shaped cell opened upward, for instance, as a cell having the depth of 1 µm and the length and width of 100 µm. However, the reaction area is not limited to the illustrated shape and size.

An end face 13a of the above-described cantilever 13 in the reaction area 10 side is surface treated so that the end part of the detecting nucleotide chain D is fixed by a chemical bond such as a coupling reaction. That is, this end face 13a may be surface treated preferably suitably for fixing the previously processed end part of the detecting nucleotide chain D such as a DNA probe. For instance, when the end face 13a of the cantilever 13 is surface treated with streptoavidin, the surface treated end face 13a is suitable for fixing the end part of the biotinylated nucleotide chain. Here, the end face 13a is surface treated, however, the end face is not necessarily limited thereto and at least a part of the cantilever 13 may be surface treated.

Here, in the nucleotide chain, an ionic cloud is considered to be formed by phosphoric ions (negative charges) that forms a skeleton of the nucleotide chains and hydrogen ions (positive charges) produced by ionizing water existing in the periphery thereof. A polarization vector generated by these negative charges and positive charges is entirely directed to one direction by applying high frequency high voltage. As a result, the nucleotide chains are stretched. Further, when an uneven electric field that a line of electric force is partly concentrated is applied to the nucleotide chains, the nucleotide chains move toward a part to which the line of electric force is concentrated (refer to Seiichi Suzuki, Takeshi Yamanashi, Shin-ichi Tazawa, Osamu Kurosawa and Masao Washizu: "Quantitative analysis on electrostatic orientation of DNA in stationary AC electric field using fluorescence anisotropy", IEEE Transaction on Industrial Application, Vol. 34, No. 1, p.75-83 (1998)).

Thus, when the electric field of high frequency high voltage that the line of electric force is concentrated to the end face 13a of each cantilever 13 is applied to the nucleotide chains by the cathode 11 and the anode 12, the nucleotide chains stretched by applying the high frequency high voltage move toward the end face 13a. Thus, the end part comes into contact with the end face 13a, so that the end part of the detecting nucleotide chain D can be assuredly fixed to the end face 13a of the cantilever 13. Especially, when a ferrodielectric member having a piezoelectricity such as PZT (lead zirconate titanate) or SBT (strontium bismuth tantalate) is employed as described below, the line of electric force is preferably concentrated to the end face 13a of the cantilever 13 due to the ferrodielectric member.

A nozzle 3 having its end part shown in Fig. 2 serves to drip sample solution S (S'). The sample solution S including the detecting nucleotide chains D and the sample solution S' including the target nucleotide chains T are dripped by precisely following the reaction area 10 on the basis of positional information and rotation synchronizing information provided from the substrate 1 as described above.

As dripping means, an ink jet printing method can be preferably employed, because very small droplets can be precisely dripped on a prescribed reaction area 10.

The "ink jet printing method" is a method to which a nozzle used in an ink jet printer is applied. The sample solution S (S') is jetted to the substrate 1 from a printer head like the ink jet printer by using electric power. This method includes a piezoelectric ink jet method, a bubble jet (registered trademark) method and a ultrasonic jet method.

The piezoelectric ink jet method is a method for spattering liquid droplets under the pressure of a displacement generated by applying a pulse to a piezoelectric member. Further, the bubble jet (registered trademark) method is a thermal method in which liquid droplets are spattered under the pressure of bubbles generated by heating a heater in a nozzle. A silicon base serving as the heater is embedded in the nozzle and controlled at about 300 °C/s to form uniform bubbles and push out them. However, the sample solution S(S') is exposed to high temperature. Accordingly, when a biological material sample is used, an attention needs to be paid. The ultrasonic jet method is a method that a ultrasonic beam is applied to a free surface of the sample solution S (S') to locally apply high pressure and eject small droplets from that part. Thus, a nozzle is not necessary and the small droplets having a diameter of about 1 µm can be formed at high speed.

In this embodiment, as the "ink jet print method", the "piezoelectric ink jet method" can be preferably employed. Since the form of the applied pulse is changed so that the size of the liquid droplets (very small droplets) can be controlled. Thus, this method is preferable for improving analysis accuracy. That is, when the radius of curvature of the surface of the liquid droplet is small, the size of the liquid droplet can be decreased. When the radius of curvature of the liquid droplet is large, the size of the liquid droplet can be increased. Further, the pulse can be abruptly changed to a negative direction to pull the surface of the droplet inward so that the radius of curvature can be reduced.

Here, most of the detecting nucleotide chain D dripped on the reaction area 10 are fixed to the end face 13a of the cantilever 13 while bases are laminated thereon. Thus, a steric hindrance is undesirably occurs upon hybridization reaction of the target nucleotide chains T dripped afterward and the detecting nucleotide chain D.

Thus, in this embodiment, a potential difference is generated between the cathode 11 and the anode 12 disposed in the cell detecting part 2 to form an electric field in a liquid layer (salt solution) stored and held in the reaction area 10. Then, the detecting nucleotide chain D is stretched in the form of a straight chain along the orientation of the electric field. The above-described high frequency high voltage is considered to be preferably located in the vicinity of 1 × 10⁶ V/m and about 1 MHz (refer to Masao Washizu and Osamu Kurosawa: "Electrostatic Manipulation of DNA in Microfabricated Structures", IEEE Transaction on Industrial Application, Vol. 26, No. 26, p.1165-1172 (1990)).

When the cell detecting parts 2 are arranged in rows on the substrate 1, the cathode 11 and the anode 12 may be provided for each of the cell detecting parts 2. Further, the cathode 11 and the anode 12 may be respectively formed in a common electrode.

As described above, the high frequency high voltage is applied to the cell detecting part 2 as described above, the detecting nucleotide chains D and the target nucleotide chains T are stretched in the forms of straight chains. Thus, the steric hindrance rarely occurs and the hybridization reaction is efficiently accelerated. As a result, the reaction time of the hybridization is shortened and a probability showing the false positive or the false negative is also decreased.

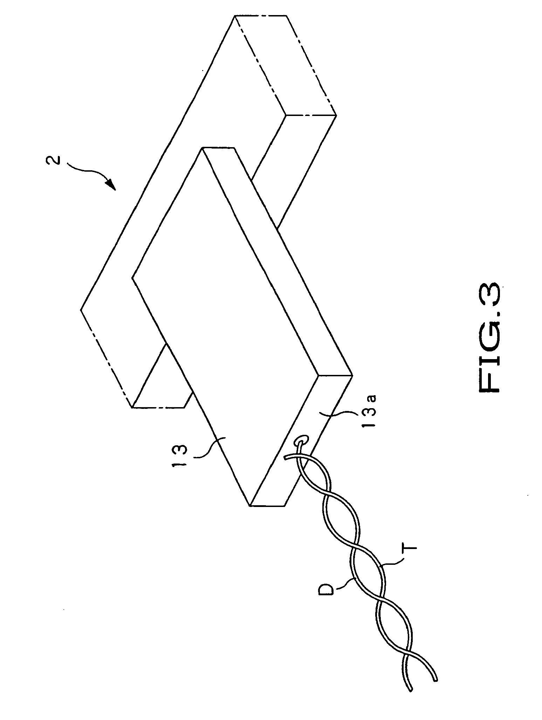

The cantilever 13 protruding to the reaction area 10 side of the cell detecting part 2 is shown in Fig. 3 in its enlarged form. Fig. 3 schematically shows a state that the detecting nucleotide chains D the end part of which is fixed to the end face 13a of the cantilever 13 are hybridized with the target nucleotide chains T having a basic arrangement complementary to the basic arrangement of the detecting nucleotide chains D to form two chains. For the purpose of simplicity, Fig. 3 shows the state that one detecting nucleotide chain D is fixed to the end face 13a. However, actually, the adequate number of the detecting nucleotide chains D are fixed to the end face depending on the width of the cantilever 13.

The reaction area 10 may be filled with a phase-change material (not shown in the drawings) such as agarose gel in which a reversible phase-change of gel and sol may occur between room temperature and suitable temperature to a reaction. In this case, a procedure can be advantageously carried out in which the phase-change material is changed to sol under high temperature, under this state, voltage is applied to the phase-change material to arrange the nucleotide chain such as a DNA of one chain, then, the temperature is lowered to change the phase-change material to the gel, and subsequently, the phase-change material is changed to the sol at the suitable temperature to the reaction upon hybridization. Further, when the phase-change material is held in the state of the gel upon hybridization, while the nucleotide chains such as the DNA are stretched, the hybridization can be preferably carried out.

Under the state that the phase-change material is changed to the gel, when the sample solution S' including the target nucleotide chains T is dripped on the cell detecting part 2, the progress of the hybridization may be probably prevented. Accordingly, a vertical groove may be previously formed at a dripping point like a case of an ordinary electrophoresis and the sample solution S' may be dripped in this groove.

The above-described cantilever 13 has a vibrator composed of a piezoelectric material disposed between the counter electrodes. AC voltage is applied to the vibrator to vibrate the vibrator in accordance with a frequency. At this time, the cantilever 13 may be entirely formed as a piezoelectric vibrator. Further, a piezoelectric element may be formed on the cantilever 13.

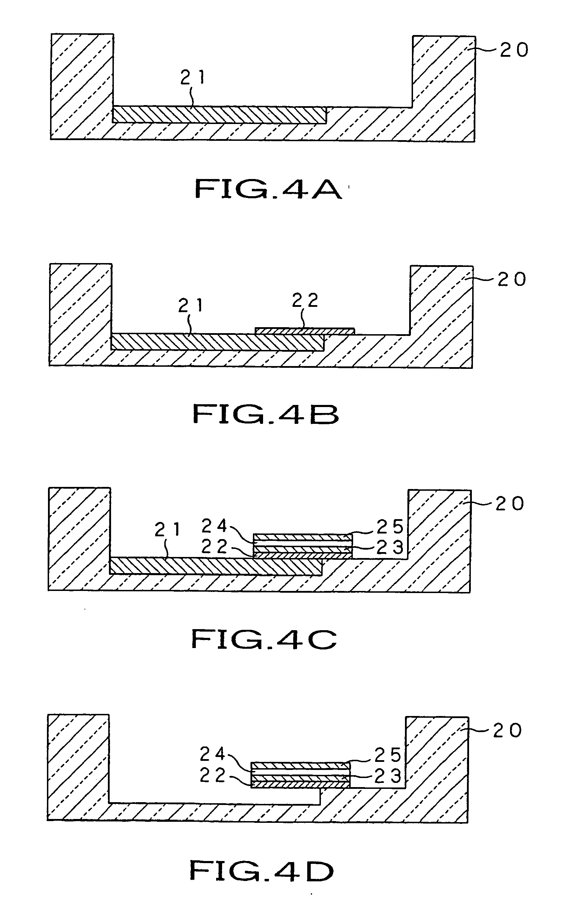

The cantilever 13 can be manufactured by utilizing a technique disclosed in, for instance, Japanese Patent Application Laid-Open No. 2001-347499. A principle of this manufacturing method is briefly described by using Figs. 4A to 4D.

Firstly, as shown in Fig. 4A, a polysilicon layer 21 serving as a sacrifice layer for finally forming a cavity part at a part of a bottom part of a silicon base 20 as the cell detecting part 2. The bottom part entirely has a uniform height. Then, a photoresist is applied to the surface of the bottom part to form a resist pattern for masking a part except a part in which the cantilever 13 is formed by a photolithography method or the like. After that, an insulating layer 22 made of SiO₂ (silicon dioxide) is formed as shown in Fig. 4B by a CVD (Chemical Vapor Deposition) method using, for instance, SiH₄-N₂O system. The insulating layer 22 may be made of SiNₓ (silicon nitride) by the CVD method using, for instance, not only SiO₂ but also SiH₄-NH₃.

Then, as shown in Fig. 4C, a lower electrode layer 23 made of Pt (platinum) or In (indium), a piezoelectric material layer 24 made of PZT (Pb(Ti, Zr)O₃: lead zirconate titanate) or SBT (SrBi₂Ta₂O₉: strontium bismuth tantalate), and an upper electrode layer 25 made of Pt or In are sequentially laminated and patterned.

Finally, as shown in Fig. 4D, XeF₂ (xenon difluoride) or BrF₃ (bromine trifluoride) is sublimed in a vacuum chamber and introduced to completely remove the polysilicon layer 21 by a selective etching operation.

In this embodiment, the change of the natural frequency of the cantilever 13 manufactured as described above is detected to detect whether or not there is the hybridization reaction.

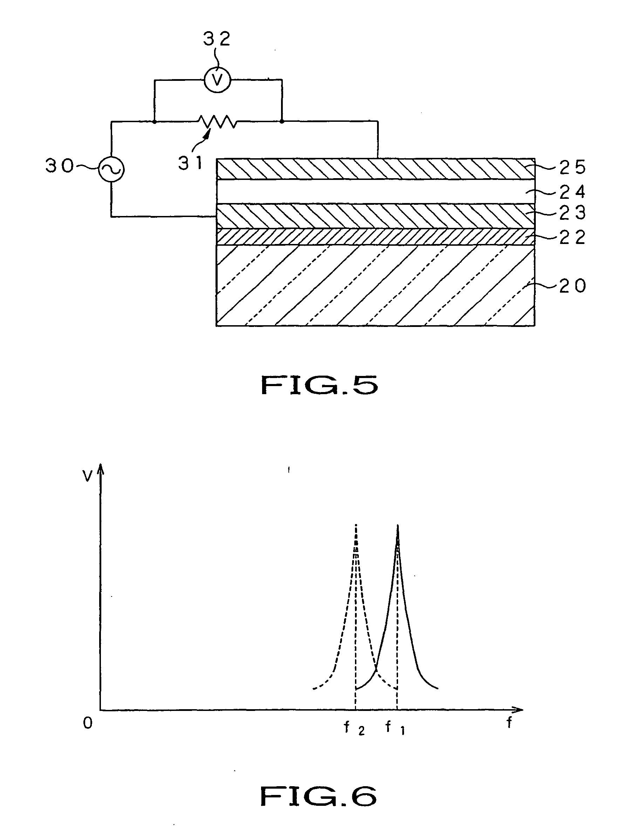

Specifically, as shown in Fig. 5, an ac circuit in which the lower electrode layer 23 is connected to the upper electrode layer 25 is formed. While frequency is changed by an ac power 30 as a driving source of the cantilever 13, the cantilever 13 is vibrated and excited. Then, electric current associated with resonance amplitude is converted to voltage by a resistance 31 and the voltage is measured by a voltmeter 32 as vibration detecting means. The waveform of the voltage measured by the voltmeter 32 is shown by, for instance, a full line in Fig. 6. Since the resonance amplitude of the cantilever 13 becomes maximum when the frequency of the ac power 30 corresponds to the natural frequency of the cantilever 13, the frequency f₁ of the ac power 30 at the time of a peak of the voltage shows the natural frequency of the cantilever 13.

Here, the detecting nucleotide chains D fixed to the end face 13a of the cantilever 13 are hybridized with the target nucleotide chains T having the basic arrangement complementary to the basic arrangement of the detecting nucleotide chains D to form two chains. In this case, the natural frequency of the cantilever 13 is lowered due to the increase of the mass of the cantilever 13 resulting from the target nucleotide chains T. Thus, the waveform of the voltage shifts downward to a position shown by a broken line in Fig. 6. The frequency of the ac power 30 at the time of a peak becomes f₂.

Accordingly, the change of the frequency (natural frequency) at the time of the peak of the voltage is detected so that whether or not there is the hybridization can be detected.

Further, since the variation (f₁-f₂) of the natural frequency is exactly correlated to the variation of the mass of the cantilever 13. Accordingly, the natural frequency of the detecting nucleotide chains D before they are fixed to the cantilever, the natural frequency of the detecting nucleotide chains D after they are fixed to the cantilever, and the natural frequency after the hybridization reaction is completed are measured so that the number of the detecting nucleotide chains D which are fixed to the cantilever 13 or the number of the target nucleotide chains T that are hybridized with the detecting nucleotide chains D can be quantitatively detected.

As the change of the mass increases, the detection sensitivity of the change of the natural frequency is more improved. Accordingly, the end of the target nucleotide chains T is preferably modified by an anchor molecule to increase its mass.

As described above, when the reaction area 10 is filled with the phase-change material such as agarose gel, the frequency cannot be probably precisely detected. Accordingly, after the hybridization reaction is completed, the phase-change material may be changed to sol before the frequency is measured and the phase-change material may be removed by an action of centrifugal force generated by rotating the substrate 1.

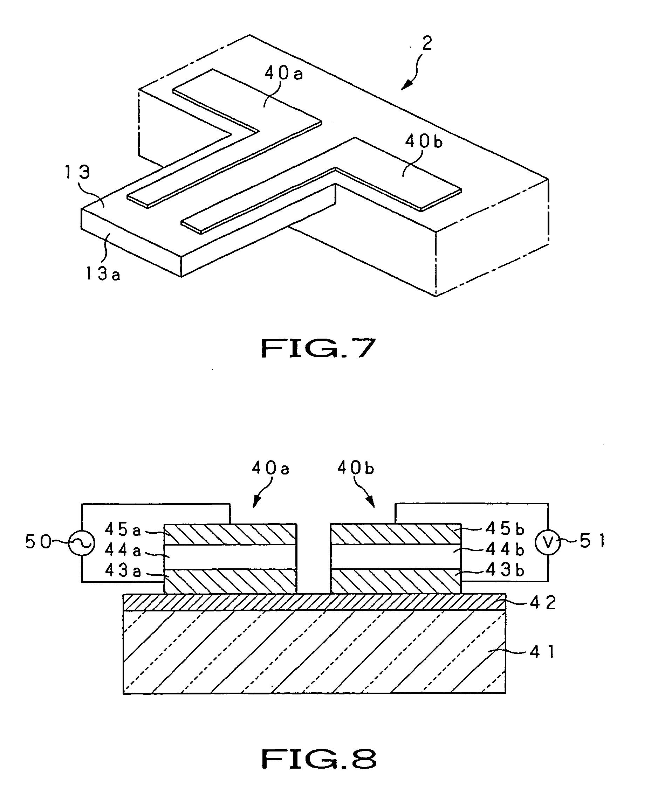

In the above-described embodiment, the entire part of the cantilever 13 is formed with the piezoelectric vibrator. However, the cantilever is not limited thereto. As shown in Fig. 7, a piezoelectric element 40a serving as a driving electrode and a piezoelectric element 40b serving as a detecting electrode may be provided on the upper part of a cantilever 13. Fig. 8 shows a vertical sectional view of the cantilever 13 at a position including the piezoelectric elements 40a and 40b in this case.

As shown in Fig. 8, in the cantilever 13, an insulating layer 42 composed of SiO₂ or SiNₓ is formed on a silicon base 41. Further, the piezoelectric elements 40a and 40b respectively include lower electrode layers 43a and 43b made of Pt or In, piezoelectric material layers 44a and 44b made of PZT or SBT, and upper electrode layers 45a and 45b made of Pt or In which are sequentially laminated.

When the presence or absence of a hybridization reaction is detected, while a frequency is changed by an ac power 50 serving as a driving source, the cantilever 13 is vibrated and excited. Then, a potential difference based on resonance amplitude is measured by a voltmeter 51 as a vibration detecting means. When the mass of the cantilever 13 is increased by the hybridization reaction as described above, the natural frequency of the cantilever 13 is lowered to decrease the frequency of the ac power 50 at the time of a peak of voltage.

When the change of the natural frequency resulting from the hybridization reaction is recognized in such a way, a fact can be recognized that target nucleotide chains complementary to detecting nucleotide chains D including marker genes related to a specific illness are present in sample solution S' extracted from, for instance, a prescribed cell. As a result, the fact that the above-described illness occurs in the cell is anticipated.

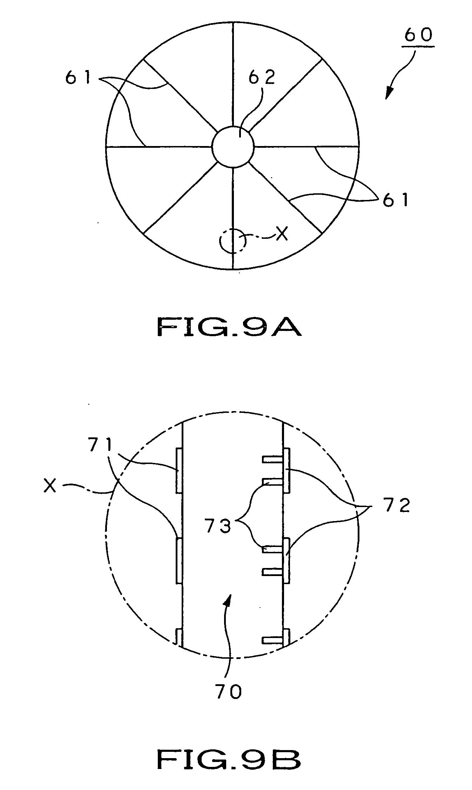

Now, another example of the substrate used for the bioassay device in this embodiment will be described by referring to Figs. 9A and 9B. Fig. 9A shows a plan view of the substrate in this example viewed from above. Fig. 9B shows an enlarged plan view of an X part in Fig. 9A.

The substrate 60 shown in Fig. 9A includes line groove detecting parts 61. In the line groove detecting part 61, a reaction area 70 has line grooves extended in radial directions on the substrate 60. From one side of a longitudinal direction forming the line groove, cantilevers 73 respectively having the same structure as that of the cantilever 13 in the cell detecting part 2 protrude at prescribed intervals as shown in Fig. 9B. In parts in which the cantilevers 73 are provided, cathodes and anodes 71 and 72 are respectively opposed so as to sandwich the reaction area 70 in between them. The line groove detecting part 61 may be considered to have a structure that the cell detecting parts 2 are arranged in the line groove detecting part 61.

Each cathode 71 may be formed as a common electrode. Each anode 72 may be also formed as a common electrode. That is, the common cathode and the common anode can be opposed to each other so as to sandwich the reaction area 70 in between them and arranged in radial directions on the substrate 60. According to this structure, a needle type probe can be applied to the common cathode and the common anode from above to supply electric current.

The above-described reaction area 70 may be formed in pits (not shown in the drawings) arranged in each line groove. Very small droplets are dripped in the reaction areas of the pits so that the substantially same spot sizes can be realized.

Further, in the line groove detecting part 61, a liquid supply means utilizing a capillary action or a liquid supplying means using a centrifugal force generated by rotating the disc shaped substrate 60 by a prescribed method can be used.

Specifically, a liquid storage part 62 is provided in the central part of the substrate 60. The sample solution S (S') or cleaning solution for removing surplus target nucleotide chains T which are not actively bonded after the reaction is injected to the liquid storage part 62. The substrate 60 is rotated to smoothly and assuredly supply the liquid to the line grooves (that is, the reaction areas 70) from the central area of the substrate.

In the line groove detecting parts 61, different detecting nucleotide chains D can be fixed to units of the line groove detecting parts or units of the grouped line groove detecting parts.

Now, the positional information and the rotating synchronizing information of the substrates 1 and 60 will be briefly described below. In the rotating directions of the substrates 1 and 60, many address pits previously formed by a disc mastering process are formed. When the substrates 1 and 60 are considered as optical discs, the reaction areas 10 and 70 as drip detecting positions may be considered to be user data areas. In other areas, synchronizing pits are arranged by a sample servo system and also used as a tracking servo. Immediately after them, address parts (geographical addresses on the disc) are inserted to give the positional information.

The address parts are composed of a combination of a sector mark starting as a first pattern, a VFO for applying the rotation phase of an actually rotating disc, an address mark for applying the start position of address data, and IDs (Identifier) including the numbers of tracks and sectors, etc.

When a bioassay is carried out by using the substrates 1 and 60 having the above-described structures, a device is used that includes at least below-described means and mechanisms. That is, the device (not shown in the drawings) at least includes a substrate rotating means for holding the substrates 1 and 60 so as to freely rotate, a dripping means for dripping the sample solution S (S') to the reaction areas 10 and 70 in prescribed order and timing while the substrates 1 and 60 are rotated by the substrate rotating means, a focus servo mechanism for holding a distance between the dripping means (a nozzle thereof) and the substrates to a prescribed distance, and a tracking servo mechanism for allowing the drip of the sample solution S (S') to follow the reaction areas 10 and 70 of the substrates 1 and 60 on the basis of positional information and rotation synchronizing information provided from the substrates 1 and 60.

When the above-described substrates 1 and 60 and the bioassay device using them are employed, the above-described interaction detecting method according to the present invention can be preferably put into practice.

That is, when the detecting nucleotide chains D fixed to the end faces of the cantilevers 13 and 73 are hybridized with the target nucleotide chains T, the masses of the cantilevers 13 and 73 are increased. Accordingly, the natural frequency of the cantilevers 13 and 73 is lowered. Thus, the change of the natural frequency is measured so that the hybridization can be detected.

Further, electric fields are formed on the reaction areas 10 and 70 by the cathodes 11 and 71 and the anodes 12 and 72. Thus, while the detecting nucleotide chains D and the target nucleotide chains T in the reaction areas 10 and 70 are stretched, they can be relative moved and hybridized. Accordingly, an efficiency of hybridization is improved.

The present invention is not limited to the above-described embodiment explained by referring to the drawings. It is to be understood to a person with ordinary skill in the art that various changes, substitutions or equivalence thereto may be made without departing the attached claims and the gist thereof.

For instance, in the above-described embodiment, the substrate for bioassay is formed in the shape of the disc. However, the substrate is not limited to this form, and a rectangular shape and other flat plate shapes may be suitably selected and determined.

The present invention is particularly preferable for the bioassay method based on the DNA chips and can be employed for the analysis of the mutation of genes, SNPs (single nucleotide polymorphism) analysis, the analysis of gene expression frequency or the like. The present invention can be utilized in a wide range including a drug discovery, a clinical diagnosis, pharmacogenomics, a forensic medicine and other fields.

## Claims

1. A method for detecting hybridization between nucleotide chains in a detecting part (2) that includes at least one cantilever (13) of which at least a part (13a) is surface treated so as to fix a detecting nucleotide chain (D) thereto, and a reaction area (10) for providing a place for hybridization between the detecting nucleotide chain (D) fixed to the surface treated part (13a) and a target nucleotide chain (T), said interaction detecting method comprising the steps of:
forming in the reaction area (10) a high frequency, high voltage electric field which is uneven such that the field is concentrated at the surface treated part (13a) of the cantilever (13);
vibrating and exciting the cantilever (13);
detecting the amplitude of vibration of the cantilever; and
detecting the hybridization by measuring the change of the natural frequency of the cantilever based on the hybridization.

2. A method according to claim 1, wherein the cantilever (13) comprises a vibrator having a piezoelectric material (24) disposed between counter electrodes (23,25) and a driving source (30) vibrates and excites the cantilever by applying an ac voltage between the counter electrodes.

3. A method according to claim 1 or claim 2, wherein the electric field forming step comprises forming the high frequency, high voltage electric field in the reaction area (10) and relatively moving the detecting nucleotide chain (D) and the target nucleotide chain (T) in the reaction area while they are stretched to carry out the hybridization.

4. A bioassay device having a detecting part (2) that comprises:
at least one cantilever (13) of which at least a part (13a) is surface treated so as to fix a detecting nucleotide chain (D) thereto;
a reaction area (10) for providing a place for hybridization between the detecting nucleotide chain (D) fixed to the surface treated part (13a) and a target nucleotide chain (T);
a driving source (30) for vibrating and exciting the cantilever;
a vibration detecting means (31, 32) for detecting the amplitude of vibration of the cantilever; and **characterised by**
an electric field forming means (11, 12, 13, V) for forming in the reaction area (10) a high frequency, high voltage electric field which is uneven such that the field is concentrated at the surface treated part (13a) of the cantilever (13).

5. A device according to claim 4, wherein the cantilever (13) comprises a vibrator having a piezoelectric material (24) disposed between counter electrodes (23, 25) and the driving source (30) is operative to vibrate and excite the cantilever by applying an ac voltage between the counter electrodes.

6. A device according to claim 4 or claim 5, wherein the electric field forming means (11, 12, 13, V) is operative to form the high frequency, high voltage electric field in the reaction area and to relatively move the detecting nucleotide chain (D) and target nucleotide chain (T) while the detecting nucleotide chain (D) and the target nucleotide chain (T) in the reaction area (10) are stretched to carry out the hybridization.

7. A device according to claim 4, claim 5 or claim 6, wherein the reaction area (10) is filled with a material in which a reversible phase change of gel and sol may be generated between room temperature and a suitable temperature for a reaction.

8. A device according to any one of claims 4 to 7, wherein the hybridization is detected by measuring the change of the natural frequency of the cantilever (13) based on the hybridization.

9. A substrate for bioassay having a plurality of bioassay devices according to any one of claims 4 to 8 disposed thereon, wherein the detecting part (2) of each bioassay device is a cell detecting part in which the cantilever (13) protrudes from a wall surface.

10. A substrate according to claim 9, which is formed as a disc type substrate with the cell detecting parts (2) of the bioassay devices provided in radial directions thereon as viewed from above.

11. A substrate according to claim 9, wherein the cell parts (2) of respective bioassay devices or grouped pluralities of bioassay devices comprise respective different detecting nucleotide chains (D).

12. A substrate according to claim 9, which is formed as a disc type substrate with the reaction areas (10) of the bioassay devices provided in line grooves extended in radial directions and with the cantilevers (13) protruding from one side of the line grooves.

13. A substrate according to claim 12, wherein the detecting nucleotide chains (D) differ as between respective line grooves or the grouped pluralities of line grooves.

## Patentansprüche

1. Verfahren zum Feststellen einer Hybridisierung zwischen Nukleotidketten in einem Detektierteil (2), das zumindest einen Freiträger (13), von dem zumindest ein Teil (13a) derart auf der Oberfläche behandelt ist, dass eine Detektier-Nukleotidkette (D) daran bzw. darauf angebracht ist, und einen Reaktionsbereich (10) zur Bereitstellung eines Platzes für eine Hybridisierung zwischen der an dem auf der Oberfläche behandelten Teil (13a) angebrachten Detektier-Nukleotidkette (D) und einer Ziel-Nukleotidkette (T) aufweist,
wobei das Zusammenwirkungs-Detektierverfahren die Schritte umfasst:
Bilden eines elektrischen Hochfrequenz-Hochspannungsfeldes in dem Reaktionsbereich (10), welches derart ungleichmäßig ist, dass es bei dem auf der Oberfläche behandelten Teil (13a) des Freiträgers (13) konzentriert ist,
In-Vibration-versetzen und Erregen des Freiträgers (13),
Detektieren der Amplitude der Vibration des Freiträgers
und Feststellen der Hybridisierung durch Messen der auf der Hybridisierung basierenden Änderung der Eigenfrequenz des Freiträgers.

2. Verfahren nach Anspruch 1, wobei der Freiträger (13) einen Vibrator mit einem piezoelektrischen Material (24) enthält, welches zwischen Gegenelektroden (23, 25) angeordnet ist,
und wobei eine Ansteuerquelle (30)den Freiträger durch Anlegen einer Wechselspannung zwischen den Gegenelektroden in Vibration versetzt und erregt.

3. Verfahren nach Anspruch 1 oder 2, wobei der Schritt zur Bildung des elektrischen Feldes die Bildung des elektrischen Hochfrequenz-Hochspannungsfeldes in dem Reaktionsbereich (10) und eine relative Bewegung der Detektier-Nukleotidkette (D) und der Ziel-Nukleotidkette (T) in dem Reaktionsbereich umfasst, während diese gestreckt werden, um die Hybridisierung auszuführen.

4. Bioassay-Vorrichtung mit einem Detektierteil (2), das umfasst:
zumindest einen Freiträger (13), von dem zumindest ein Teil (13a) auf der Oberfläche derart behandelt ist, dass an diesem bzw. auf dieser eine Detektier-Nukleotidkette (D) angebracht ist,
einen Reaktionsbereich (10) zur Bereitstellung eines Platzes für eine Hybridisierung zwischen der an dem auf der Oberfläche behandelten Teil (13a) angebrachten Detektier-Nukleotidkette (D) und einer Ziel-Nukleotidkette (T),
eine Ansteuerquelle (30), die den Freiträger in Vibration versetzt und erregt,
und eine Vibrations-Detektiereinrichtung (31, 32) zum Ermitteln der Amplitude der Vibration des Freiträgers,
**gekennzeichnet durch** eine ein elektrisches Feld bildende Einrichtung (11, 12, 13, V) zur Bildung eines elektrischen Hochfrequenz-Hochspannungsfeldes in dem Reaktionsbereich (10), welches derart ungleichmäßig ist, dass es bei dem auf der Oberfläche behandelten Teil (13a) des Freiträgers (13) konzentriert ist.

5. Vorrichtung nach Anspruch 4, wobei der Freiträger (13) einen Vibrator mit einem piezoelektrischen Material (24) enthält, welches zwischen Gegenelektroden (23, 25) angeordnet ist,
und wobei die Ansteuerquelle (30) derart betrieben ist, um den Freiträger durch Anlegen einer Wechselspannung zwischen den Gegenelektroden in Vibration zu versetzen und zu erregen.

6. Vorrichtung nach Anspruch 4 oder 5, wobei die das elektrische Feld bildende Einrichtung (11, 12, 13, V) derart betrieben ist, dass das elektrische Hochfrequenz-Hochspannungsfeld in dem Reaktionsbereich gebildet ist und dass die Detektier-Nukleotidkette (D) sowie die Ziel-Nukleotidkette (T) relativ bewegt werden, während die Detektier-Nukleotidkette (D) und die Ziel-Nukleotidkette (T) in dem Reaktionsbereich (10) gestreckt werden, um die Hybridisierung auszuführen.

7. Vorrichtung nach Anspruch 4, 5 oder 6, wobei der Reaktionsbereich (10) mit einem Material gefüllt ist, in welchem eine reversible Phasenänderung eines Gels eines Sols zwischen Zimmertemperatur und einer für eine Reaktion geeigneten Temperatur hervorgerufen werden kann.

8. Vorrichtung nach einem der Ansprüche 4 bis 7, wobei die Hybridisierung durch Messen der auf der Hybridisierung basierenden Änderung der Eigenfrequenz des Freiträgers (13) festgestellt wird.

9. Substrat für ein Bioassay, auf dem eine Mehrzahl von Bioassay-Vorrichtungen nach einem der Ansprüche 4 bis 8 angeordnet ist, wobei das Detektierteil (2) jeder Bioassay-Vorrichtung ein Zell-Detektierteil ist, in welchem der Freiträger (13) von einer Wandfläche absteht.

10. Substrat nach Anspruch 9, welches als Substrat vom Disktyp gebildet ist, auf dem die Zell-Detektierteile (2) der Bioassay-Vorrichtungen bei Betrachtung von oben in radialen Richtungen vorgesehen sind.

11. Substrat nach Anspruch 9, wobei die Zellteile (2) der Bioassay-Vorrichtungen oder einer gruppierten Mehrzahl von Bioassay-Vorrichtungen jeweils unterschiedliche Detektier-Nukleotidketten (D) enthalten.

12. Substrat nach Anspruch 9, welches als Substrat vom Disktyp gebildet ist, bei dem die Reaktionsbereiche (10) der Bioassay-Vorrichtungen in Reihennuten vorgesehen sind, welche in radialen Richtungen verlaufen, wobei die Freiträger (13) von einer Seite der Reihennuten vorstehen.

13. Substrat nach Anspruch 12, wobei die Detektier-Nukleotidketten (D) sich zwischen den jeweiligen Reihennuten oder den gruppierten Mehrzahlen von Reihennuten unterscheiden.

## Revendications

1. Procédé pour détecter une hybridation entre des chaînes nucléotidiques dans une partie de détection (2) qui inclut au moins une console (13) dont au moins une partie (13a) est traitée en surface de manière à fixer à celle-ci une chaîne nucléotidique de détection (D), et une zone de réaction (10) pour former un site pour l'hybridation entre la chaîne nucléotidique de détection (D) fixée à la partie traitée en surface (13a) et une chaîne nucléotidique cible (T), ledit procédé de détection d'interaction comprenant les étapes de :
former dans la zone de réaction (10) un champ électrique à haute tension et haute fréquence qui est non uniforme de sorte que le champ est concentré au niveau de la partie traitée en surface (13a) de la console (13) ;
faire vibrer et exciter la console (13) ;
détecter l'amplitude de vibration de la console ; et
détecter l'hybridation en mesurant le changement de la fréquence propre de la console basé sur l'hybridation.

2. Procédé selon la revendication 1, où la console (13) comprend un vibreur ayant un matériau piézoélectrique (24) disposé entre des contre-électrodes (23, 25) et une source de commande (30) fait vibrer et excite la console par application d'une tension alternative entre les contre-électrodes.

3. Procédé selon la revendication 1 ou la revendication 2, où l'étape de formation du champ électrique comprend la formation du champ électrique à haute tension et haute fréquence dans la zone de réaction (10) et le déplacement relatif de la chaîne nucléotidique de détection (D) et de la chaîne nucléotidique cible (T) dans la zone de réaction tandis qu'elles sont étirées pour réaliser l'hybridation.

4. Dispositif de biotest ayant une partie de détection (2) qui comprend :
au moins une console (13) dont au moins une partie (13a) est traitée en surface de manière à fixer à celle-ci une chaîne nucléotidique de détection (D) ;
une zone de réaction (10) pour former un site pour l'hybridation entre la chaîne nucléotidique de détection (D) fixée à la partie traitée en surface (13a) et une chaîne nucléotidique cible (T) ;
une source de commande (30) pour faire vibrer et exciter la console ;
un moyen de détection de vibration (31, 32) pour détecter l'amplitude de vibration de la console, **caractérisé par**
un moyen formant un champ électrique (11, 12, 13, V) pour former dans la zone de réaction (10) un champ électrique à haute tension et haute fréquence qui est non uniforme de sorte que le champ est concentré au niveau de la partie traitée en surface (13a) de la console (13).

5. Dispositif selon la revendication 4, où la console (13) comprend un vibreur ayant un matériau piézoélectrique (24) disposé entre des contre-électrodes (23, 25) et la source de commande (30) est active pour faire vibrer et exciter la console par application d'une tension alternative entre les contre-électrodes.

6. Dispositif selon la revendication 4 ou la revendication 5, où le moyen formant un champ électrique (11, 12, 13, V) est actif pour former le champ électrique à haute tension et haute fréquence dans la zone de réaction et pour déplacer relativement la chaîne nucléotidique de détection (D) et la chaîne nucléotidique cible (T) tandis que la chaîne nucléotidique de détection (D) et la chaîne nucléotidique cible (T) dans la zone de réaction (10) sont étirées pour réaliser l'hybridation.

7. Dispositif selon la revendication 4, la revendication 5 ou la revendication 6, où la zone de réaction (10) est remplie d'un matériau dans lequel un changement de phase réversible de gel et de sol peut être produit entre la température ambiante et une température appropriée pour une réaction.

8. Dispositif selon l'une quelconque des revendications 4 à 7, où l'hybridation est détectée en mesurant le changement de la fréquence propre de la console (13) basé sur l'hybridation.

9. Substrat pour biotest ayant une pluralité de dispositifs de biotest selon l'une quelconque des revendications 4 à 8 disposés sur lui, où la partie de détection (2) de chaque dispositif de biotest est une partie de détection cellulaire où la console (13) fait saillie d'une surface de paroi.

10. Substrat selon la revendication 9, qui est formé sous forme d'un substrat de type disque avec les parties de détection cellulaires (2) des dispositifs de biotest disposées dans des directions radiales sur lui en vue de dessus.

11. Substrat selon la revendication 9, où les parties cellulaires (2) de dispositifs de biotest respectifs ou de pluralités groupées de dispositifs de biotest comprennent des chaînes nucléotidiques de détection (D) différentes respectives.

12. Substrat selon la revendication 9, qui est formé sous forme d'un substrat de type disque avec les zones de réaction (10) des dispositifs de biotest disposées dans des rainures linéaires qui s'étendent dans des directions radiales et avec les consoles (13) en saillie d'un côté des rainures linéaires.

13. Substrat selon la revendication 12, où les chaînes nucléotidiques de détection (D) diffèrent entre des rainures linéaires respectives ou les pluralités groupées de rainures linéaires.
